(19) Europäisches Patentamt / European Patent Office / Office européen des brevets

(11) EP 3 639 754 A1

(12) EUROPEAN PATENT APPLICATION
published in accordance with Art. 153(4) EPC

(43) Date of publication:
22.04.2020 Bulletin 2020/17

(51) Int Cl.:
A61B 8/14 (2006.01)        A61B 5/055 (2006.01)
A61B 6/03 (2006.01)        A61B 8/13 (2006.01)

(21) Application number: 18816978.3

(22) Date of filing: 06.06.2018

(86) International application number:
PCT/JP2018/021670

(87) International publication number:
WO 2018/230409 (20.12.2018 Gazette 2018/51)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 15.06.2017  JP 2017118013

(71) Applicant: Canon Kabushiki Kaisha
Tokyo 146-8501 (JP)

(72) Inventor: KATO, Kouichi
Tokyo 146-8501 (JP)

(74) Representative: Houle, Timothy James
Canon Europe Ltd
European Patent Department
3 The Square
Stockley Park
Uxbridge, Middlesex UB11 1ET (GB)

(54) INFORMATION PROCESSING DEVICE, INFORMATION PROCESSING METHOD, AND PROGRAM

(57)     An information processing apparatus according to the present invention acquires first to fourth volume data, generates a first tomographic image corresponding to a cross section set based on a command of a user from the first volume data, generates a second tomographic image corresponding to the first tomographic image from the second volume data, identifies a third tomographic image resembling the second tomographic image from the fourth volume data, acquires information indicating a position of the third tomographic image, generates a fourth tomographic image corresponding to the third tomographic image from the third volume data based on the information indicating the position of the third tomographic image, and causes display means to display an image including the fourth tomographic image.

FIG. 2

## Description

Technical Field

The present invention relates to information processing apparatuses that display images based on volume data obtained by modality devices.

Background Art

In medical image diagnoses performed by doctors, lesions are temporally observed. For example, a subject is photographed by a modality device before and after a drug treatment, and the doctor compares the medical images obtained before and after the treatment so as to perform temporal observation for determining whether the size of a tumor has changed.

Patent Literature 1 discloses a method for temporally observing a lesion. The method involves detecting a difference in form between a current image and a past image and converting this difference information into an image.

Citation List

Patent Literature

PTL 1: Japanese Patent Laid-Open No. 7-65146

Summary of Invention

When a treatment is typically performed, the form (size and shape) of a lesion, such as a tumor, changes and the position of the lesion changes accordingly. Specifically, because the appearance of the lesion changes before and after the treatment, identification of the lesion confirmed in the pre-treatment medical image may be difficult in the post-treatment medical image.

An object of the present invention is to provide an information processing apparatus that can perform image display that allows a lesion to be easily identifiable when the lesion is temporally observed using medical images. Solution to Problem

An information processing apparatus according to the present invention includes: volume-data acquiring means for acquiring first volume data and second volume data whose coordinate systems correspond with each other and that are of different image types, acquiring third volume data of an image type identical to that of the first volume data and whose coordinate system is different from that of the first volume data, and acquiring fourth volume data of an image type identical to that of the second volume data and whose coordinate system corresponds with that of the third volume data; tomographic-image generating means for generating a first tomographic image corresponding to a cross section set based on a command of a user from the first volume data, and generating a second tomographic image corresponding to the first tomographic image from the second volume data; positional-information acquiring means for identifying a third tomographic image resembling the second tomographic image from the fourth volume data, and acquiring information indicating a position of the third tomographic image; and display control means. The tomographic-image generating means generates a fourth tomographic image corresponding to the third tomographic image from the third volume data based on the information indicating the position of the third tomographic image. The display control means causes display means to display an image including the fourth tomographic image.

Brief Description of Drawings

[Fig. 1A] Fig. 1A schematically illustrates a display image according to a comparative example.
[Fig. 1B] Fig. 1B schematically illustrates a display image according to the comparative example.
[Fig. 2] Fig. 2 schematically illustrates ultrasonic image data and photoacoustic image data before and after a treatment in accordance with this embodiment.
[Fig. 3] Fig. 3 schematically illustrates a display image on a display unit according to this embodiment.
[Fig. 4] Fig. 4 is a block diagram illustrating the configuration of a modality device according to this embodiment.
[Fig. 5A] Fig. 5A schematically illustrates the configuration of a probe according to this embodiment.
[Fig. 5B] Fig. 5B schematically illustrates the configuration of the probe according to this embodiment.
[Fig. 6] Fig. 6 is a block diagram illustrating the configuration of a computer and its surroundings according to this embodiment.

[Fig. 7] Fig. 7 is a block diagram illustrating the flow of an information processing method according to this embodiment.

[Fig. 8A] Fig. 8A schematically illustrates a GUI according to this embodiment.

[Fig. 8B] Fig. 8B schematically illustrates the GUI according to this embodiment.

Description of Embodiments

The present invention relates to an image display method based on volume data expressing image data in a three-dimensional space.

The present invention can be applied to volume data generated in any type of modality device, such as photoacoustic image data, ultrasonic image data, MRI image data, and CT image data. Photoacoustic image data is volume data expressing a three-dimensional spatial distribution of subject information of at least one of a generated sound pressure (initial sound pressure) of a photoacoustic wave, an optical absorption energy density, an optical absorption coefficient, and concentration (such as an oxygen saturation) of a material constituting a subject.

For example, the present invention can be applied when the effect of a treatment on a lesion, such as a tumor, is to be temporally observed.

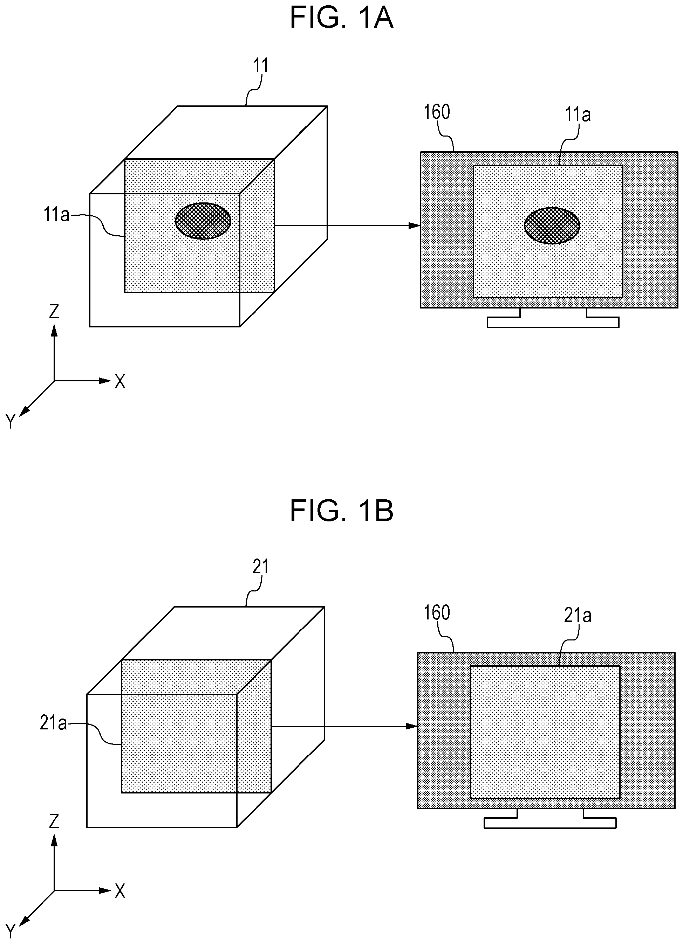

First, a comparative example where temporal observation is performed without applying the present invention will be described with reference to Fig. 1. Fig. 1A illustrates ultrasonic image data 11 obtained by an ultrasonic diagnostic device before a drug treatment. The ultrasonic image data 11 is volume data obtained in an (X, Y, Z) device coordinate system. In Fig. 1A, a tomographic image 11a corresponding to a specific XZ plane of the ultrasonic image data 11 is displayed on a display unit 160. In the comparative example, it is assumed that a tumor image as a lesion indicated in a dark color is depicted in the tomographic image 11a.

Fig. 1B illustrates ultrasonic image data 21 obtained by the ultrasonic diagnostic device after the drug treatment. Similar to the ultrasonic image data 11, the ultrasonic image data 21 is volume data obtained in the (X, Y, Z) device coordinate system. In Fig. 1B, a tomographic image 21a of the ultrasonic image data 21 corresponding to the same XZ plane as the tomographic image 11a is displayed on the display unit 160.

The position and the shape of a target to be photographed or the position and the orientation of a probe may sometimes vary before and after a treatment. Therefore, if the coordinates of the ultrasonic image data are defined with reference to the device coordinate system, the tumor image may be positioned at coordinates that are different between the pre-treatment image data and the post-treatment image data. In this description, a state where the positional relationship between the device coordinate system and the target to be photographed varies between the pieces of image data will be expressed as a state where the coordinate system varies between the pieces of image data. In other words, it can be regarded that the coordinate system of the ultrasonic image data 21 is different from the coordinate system of the ultrasonic image data 11.

The determination of whether or not there is an effect of a drug treatment is sometimes performed by observing a change in the size of the tumor before and after the treatment. In this case, the tumor may disappear by completely responding to the drug, or the tumor may significantly decrease in size. In this case, it is sometimes not possible to ascertain the position of the tumor before the treatment from the post-treatment ultrasonic image data 21.

As described above, the comparative example has the following problems. When a doctor cannot confirm a tumor image in the tomographic image 21a, the doctor sometimes cannot determine whether the tumor image does not exist because the tomographic image 21a depicts the position of the tumor different from the position thereof before the treatment, or whether the tumor image does not exist because the tumor has disappeared.

The present inventor has discovered a method for identifying the position of a lesion between pieces of volume data by referring to volume data of an image type different from ultrasonic image data. In particular, the present inventor has focused on the fact that the degree of change in the form of a blood vessel is smaller than the degree of change in the form of a tumor. Specifically, the present inventor has discovered that the position of a lesion (tumor) can be accurately identified before and after a treatment by referring to volume data that contains a blood vessel image.

For example, as volume data containing a blood vessel image, volume data of an image type, such as photoacoustic image data, MRA (magnetic resonance angiography) image data, CTA (computed tomography angiography) image data, or contrast-enhanced ultrasonic image data, may be used. As volume data containing a tumor image, volume data of an image type, such as ultrasonic image data, MRI image data, or CT image data, may be used. Volume data of any image type may be used so long as the volume data contains a blood vessel image or a tumor image.

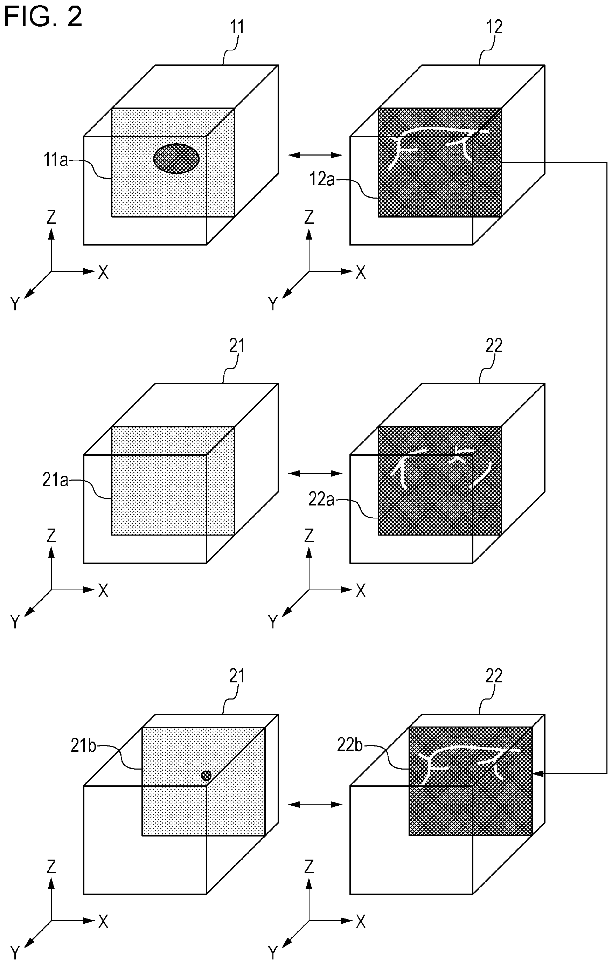

Next, an embodiment where temporal observation is performed by applying the present invention will be described with reference to Fig. 2. The following description of this embodiment relates to a case where ultrasonic image data is used as volume data containing a tumor image and photoacoustic image data is used as volume data containing a blood vessel image.

Fig. 2 schematically illustrates the ultrasonic image data and the photoacoustic image data. The ultrasonic image data 11 and photoacoustic image data 12 are obtained by a common modality device by photographing a subject at substantially the same time before a treatment, and the coordinate systems of the two pieces of image data are set in correspondence with each other. Specifically, in the ultrasonic image data 11 and the photoacoustic image data 12, a lesion is depicted at substantially the same coordinates. When the coordinate systems are set in correspondence with each other between the two pieces of image data, the position of a lesion confirmed in one of the pieces of image data can be uniquely identified in the other piece of image data.

The ultrasonic image data 21 and photoacoustic image data 22 are similarly obtained by the common modality device by photographing the subject at substantially the same time after the treatment, and the coordinate systems of the two pieces of image data are set in correspondence with each other. Specifically, in the ultrasonic image data 21 and the photoacoustic image data 22, the lesion is depicted at substantially the same coordinates. However, since the coordinate system varies before and after the treatment, it may sometimes be difficult to identify the position of the lesion between the ultrasonic image data 11 and the ultrasonic image data 21.

An example of information processing executed by an information processing apparatus according to this embodiment will be described below. A user designates the tomographic image 11a that depicts a tumor image from the pre-treatment ultrasonic image data 11. When the information processing apparatus receives the designation of the tomographic image 11a from the user, the information processing apparatus generates a tomographic image 12a of the same cross section as the tomographic image 11a from the pre-treatment photoacoustic image data 12.

Subsequently, the information processing apparatus identifies a tomographic image 22b that resembles the pre-treatment tomographic image 12a from the post-treatment photoacoustic image data 22 by image processing. Then, the information processing apparatus acquires information indicating the position of the tomographic image 22b. In this case, since the coordinate system varies before and the after the treatment, the position of the tomographic image 22b is different from the position of the tomographic image 12a. As shown in Fig. 2, in the post-treatment photoacoustic image data 22, it is comprehensible that a tomographic image 22a of the same cross section as the tomographic image 12a depicts a blood vessel different from that in the tomographic image 12a.

Subsequently, the information processing apparatus generates a tomographic image 21b of the same cross section as the tomographic image 22b (i.e., tomographic image 21b corresponding to the tomographic image 22b) from the post-treatment ultrasonic image data 21 based on the information indicating the position of the tomographic image 22b.

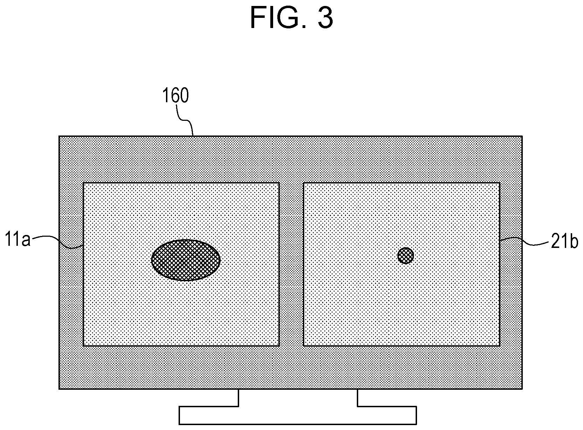

As shown in Fig. 3, the information processing apparatus can cause the display unit 160 to display the pre-treatment tomographic image 11a designated by the user and the post-treatment tomographic image 21b generated by the information processing apparatus. A tomographic image to be displayed on the display unit 160 may be deformed, such as expanded, rotated, and shifted, based on a command of the user so long as the image is generated from volume data.

As described above, it is sometimes difficult to generate a tomographic image corresponding to the lesion included in the pre-treatment tomographic image 11a directly from the ultrasonic image data 21. On the other hand, by searching for a cross section that corresponds between pieces of ultrasonic image data via the photoacoustic image data 12 and the photoacoustic image data 22 that contain a blood vessel image, the post-treatment tomographic image 21b corresponding to the pre-treatment tomographic image 11a can be generated. For example, a doctor can simply designate the tomographic image 11a including the lesion from the pre-treatment ultrasonic image data 11, so as to perform observation by comparing the image with the post-treatment tomographic image 21b including the same lesion. Accordingly, the doctor can perform temporal observation more readily, as compared with the comparative example.

Although the above embodiment relates to an example where a temporal change in a tumor as a lesion is observed, the lesion that changes over time is not limited to a tumor. For example, in order to temporally observe an inflammatory site, the information processing apparatus may acquire volume data containing an image of the inflammatory site. Moreover, although the above embodiment relates to an example where volume data containing a blood vessel image with a small temporal change relative to a lesion is acquired, the site with a small temporal change is not limited to a blood vessel. For example, the information processing apparatus may acquire volume data containing an image of at least one of a lymph vessel and a characteristic skin structure as a site with a small temporal change relative to a lesion.

Preferred embodiments of the present invention will be described below with reference to the drawings. The dimensions, materials, shapes, and relative positions of components to be described below may be changed, where appropriate, depending on the configuration of an apparatus to which the invention is applied or various conditions, and the scope of the invention should not be limited to the following description.

The following description of this embodiment relates to an example where images based on ultrasonic image data and photoacoustic image data are displayed. A modality device according to this embodiment is a multi-modality device having the functions of both an ultrasonic diagnostic device and a photoacoustic device. The configuration of the modality device and an information processing method according to this embodiment will be described below.

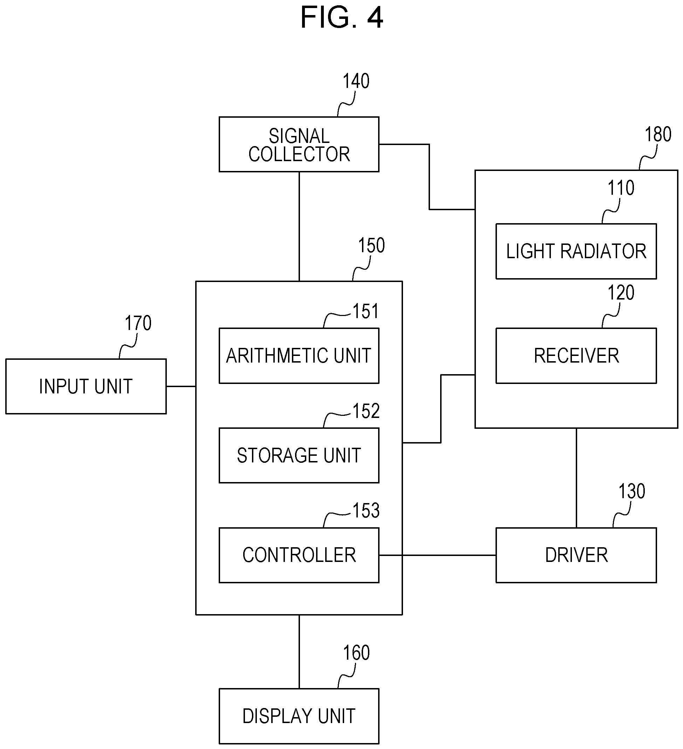

The configuration of the modality device according to this embodiment will be described with reference to Fig. 4. Fig. 4 is a schematic block diagram of the entire modality device. The modality device according to this embodiment has a probe 180 including a light radiator 110 and a receiver 120, a driver 130, a signal collector 140, a computer 150, a display unit 160, and an input unit 170.

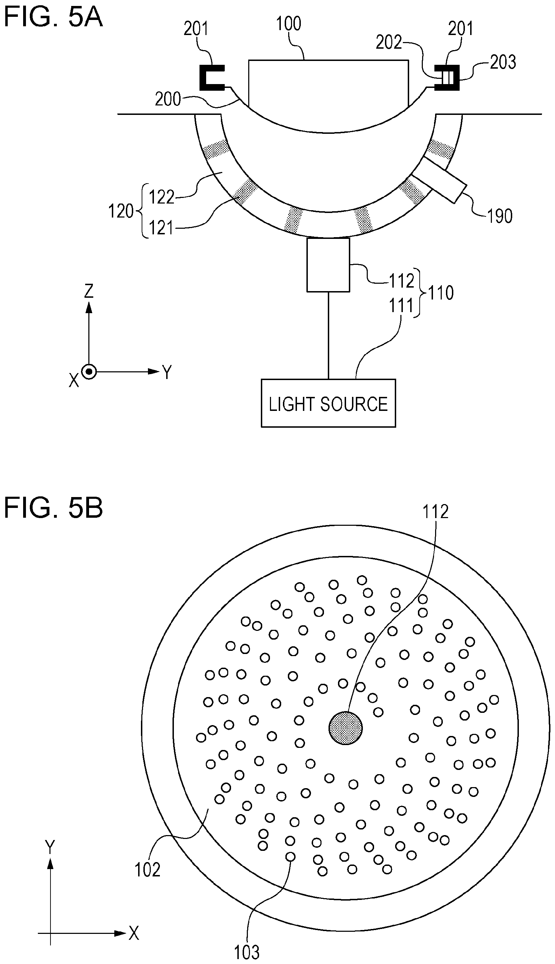

Fig. 5 schematically illustrates the probe 180 according to this embodiment. A target to be measured is a subject 100. The driver 130 drives the light radiator 110 and the receiver 120 to perform mechanical scanning. The light radiator 110 radiates light onto the subject 100, so that an acoustic wave is generated within the subject 100. An acoustic wave derived from light and generated by a photoacoustic effect is also called a photoacoustic wave. The receiver 120 outputs an electric signal (photoacoustic signal) as an analog signal by receiving a photoacoustic wave. The receiver 120 exchanges an ultrasonic wave with the subject 100 so as to output an electric signal (ultrasonic echo signal) as an analog signal.

The signal collector 140 converts the analog signal output from the receiver 120 into a digital signal and outputs the digital signal to the computer 150. The computer 150 stores the digital signal output from the signal collector 140 as signal data derived from the photoacoustic wave and the ultrasonic echo.

The computer 150 performs signal processing on the stored digital signal so as to generate volume data (photoacoustic image data and ultrasonic image data) expressing a three-dimensional spatial distribution of information related to the subject 100 (subject information). Moreover, the computer 150 causes the display unit 160 to display an image based on the volume data. A doctor who is a user can perform a diagnosis by checking the image displayed on the display unit 160. Based on a store command from the user or the computer 150, the display image is stored in a memory in the computer 150 or in a data management system connected to the modality device via a network.

The computer 150 also performs drive control on the components included in the modality device. The display unit 160 may also display a GUI in addition to the image generated by the computer 150. The input unit 170 is configured such that the user can input information thereto. The user may use the input unit 170 to perform an operation for starting or terminating a measuring process or for giving a command for storing the created image.

The components of the modality device according to this embodiment will be described in detail below.

(Light Radiator 110)

The light radiator 110 includes a light source 111 that emits light and an optical system 112 that guides the light output from the light source 111 to the subject 100. The light includes pulsed light having a so-called rectangular wave or triangular wave.

The pulse width of the light emitted by the light source 111 may range between 1 ns and 100 ns inclusive. The wavelength of the light may range from about 400 nm to 1600 nm. In a case where a blood vessel is to be imaged with high resolution, a wavelength with high absorbability (between 400 nm and 700 nm inclusive) in the blood vessel may be used. In a case where a deep area of a biological organism is to be imaged, the light used may have a wavelength with typically low absorbability (between 700 nm and 1100 nm inclusive) in background tissue (water and fat) of the biological organism.

The light source 111 used may be a laser or a light-emitting diode. When the measuring process is to be performed by using light with a plurality of wavelengths, a wavelength-variable light source may be used. If a plurality of wavelengths are to be radiated onto the subject, a plurality of light sources that generate light beams having different wavelengths may be prepared, and the light beams may be alternately radiated from the respective light sources. If a plurality of light sources are used, they are collectively expressed as a light source. A laser used may be any of various lasers, such as a solid-state laser, a gas laser, a dye laser, or a semiconductor laser. For example, a pulsed laser, such as a Nd:YAG laser or an alexandrite laser, may be used as the light source. Alternatively, a Ti:sa laser or OPO (optical parametric oscillator) laser using Nd:YAG laser light as excitation light may be used as the light source. As another alternative, a flash lamp or a light-emitting diode may be used as the light source 111. As another alternative, a microwave source may be used as the light source 111.

The optical system 112 used may include optical elements, such as a lens, a mirror, and an optical fiber. In a case where the subject 100 is, for example, a breast, the light emitter of the optical system may be constituted by, for example, a diffuser that diffuses light so that pulsed light with an increased beam diameter can be radiated. On the other hand, in a photoacoustic microscope, the light emitter of the optical system 112 may be constituted by, for example, a lens to increase the resolution, so that a beam can be radiated in a focused state.

Instead of being equipped with the optical system 112, the light radiator 110 may radiate light directly onto the subject 100 from the light source 111.

(Receiver 120)

The receiver 120 includes a transducer 121 that outputs an electric signal by receiving an acoustic wave, and also includes a supporter 122 that supports the transducer 121. The transducer 121 may also serve as transmitting means that transmits an acoustic wave. A transducer serving as receiving means and a transducer serving as transmitting means may be a single (common) transducer or may be separate components.

The transducer 121 may be composed of, for example, a piezoelectric ceramic material as typified by PZT (lead zirconate titanate) or a high-polymer piezoelectric film material as typified by PVDF (polyvinylidene fluoride). Alternatively, an element other than a piezoelectric element may be used. For example, a capacitive micro-machined ultrasonic transducer (CMUT) or a transducer that uses a Fabry-Perot interferometer may be used. Any transducer may be employed so long as it can output an electric signal by receiving an acoustic wave. A signal that is obtained by a transducer is a time-resolved signal. Specifically, the amplitude of a signal obtained by a transducer indicates a value based on a sound pressure received by the transducer at each time point (e.g., a value proportional to the sound pressure).

Frequency components constituting a photoacoustic wave typically range from 100 KHz to 100 MHz, and the transducer 121 that can be employed is capable of detecting these frequencies.

The supporter 122 may be composed of, for example, a metallic material with high mechanical strength. In order to cause a large amount of radiated light to enter a subject, the surface of the supporter 122 closer toward the subject 100 may be processed to be a mirror-finished surface or a light-scattering surface. In this embodiment, the supporter 122 has the shape of a hemispherical shell and can support a plurality of transducers 121 on the hemispherical shell. In this case, the directional axes of the transducers 121 disposed on the supporter 122 converge near the center of curvature of the hemisphere. When an image is formed by using signals output from the plurality of transducers 121, the image quality near the center of curvature becomes high. The supporter 122 may have any configuration so long as it can support the transducers 121. On the supporter 122, the plurality of transducers may be arranged in a flat surface or curved surface called a one-dimensional array, a 1.5-dimensional array, a 1.75-dimensional array, or a 2-dimensional array. The plurality of transducers 121 correspond to a plurality of receiving means.

The supporter 122 may also function as a container that stores an acoustic matching material 210. Specifically, the supporter 122 may serve as a container for disposing the acoustic matching material 210 between the transducers 121 and the subject 100.

The receiver 120 may include an amplifier that amplifies time-series analog signals output from the transducers 121. Moreover, the receiver 120 may include an A/D converter that converts the time-series analog signals output from the transducers 121 into time-series digital signals. Specifically, the receiver 120 may include the signal collector 140 to be described later.

In order to detect an acoustic wave at various angles, the transducers 121 may ideally be disposed to entirely surround the subject 100. However, if the transducers cannot be disposed to entirely surround the subject 100 due to the subject 100 being large, a state similar thereto may be achieved by disposing the transducers on the hemispherical supporter 122 in an entirely surrounding fashion.

The receiver 120 may be of a handheld type that includes a gripper. Moreover, the receiver 120 may be of a mechanically-scanning type that includes a driver for mechanically moving the transducers 121.

The arrangement and the number of transducers and the shape of the supporter may be optimized in accordance with the subject. With regard to the present invention, any type of receiver 120 may be employed.

The space between the receiver 120 and the subject 100 is filled with a medium that allows a photoacoustic wave to propagate therethrough. The medium used is a material that allows an acoustic wave to propagate therethrough, has matching acoustic characteristics at the interface between the subject 100 and the transducers 121, and has high transmittance for a photoacoustic wave as much as possible. For example, water or ultrasound gel may be used as this medium.

Fig. 5A is a side view of the probe 180, and Fig. 5B is a top view of the probe 180 (as viewed from above the plane of drawing in Fig. 5A). The probe 180 according to this embodiment shown in Fig. 5 has the receiver 120 in which the plurality of transducers 121 are three-dimensionally disposed on the hemispherical supporter 122 having an opening. Furthermore, in the probe 180 shown in Fig. 5, the light emitter of the optical system 112 is disposed at the bottom of the supporter 122.

As shown in Fig. 5, in this embodiment, the subject 100 comes into contact with a retainer 200 so that the shape of the subject 100 is maintained. In this embodiment, if the subject 100 is a breast, a bed that supports the subject person in a prone position is provided with an opening for fitting the breast therein, so that the breast hanging in the vertical direction through the opening can be measured.

The space between the receiver 120 and the retainer 200 is filled with a medium (acoustic matching material 210) that allows a photoacoustic wave to propagate therethrough. The medium used is a material that allows a photoacoustic wave to propagate therethrough, has matching acoustic characteristics at the interface between the subject 100 and the transducers 121, and has high transmittance for a photoacoustic wave as much as possible. For example, water or ultrasound gel may be used as this medium.

The retainer 200 as retaining means is used for maintaining the shape of the subject 100 during a measuring process. By using the retainer 200 to retain the subject 100, movement of the subject 100 can be suppressed and the subject 100 can be positionally kept within the retainer 200. The retainer 200 may be composed of a resin material, such as polycarbonate, polyethylene, or polyethylene terephthalate.

The retainer 200 is preferably composed of a material having enough hardness for retaining the subject 100. The retainer 200 may be composed of a material that allows light used in the measuring process to be transmitted therethrough. The retainer 200 may be composed of a material with an impedance that is about the same as that of the subject 100. If the subject 100 has a curved surface of, for example, a breast, the retainer 200 may be shaped to have a recessed shape. In this case, the subject 100 can be fitted in the recess of the retainer 200.

The retainer 200 is attached to an attachment section 201. The attachment section 201 may be configured such that a plurality of types of retainers 200 are replaceable in conformity with the size of the subject. For example, the attachment section 201 may be configured to allow for replacement of retainers with various radii of curvature and various centers of curvature.

The retainer 200 may have a tag 202 with information about the retainer 200 registered therein. Examples of the information that can be registered in the tag 202 include the radius of curvature and the center of curvature of the retainer 200, the sound velocity, and an identification ID. The information registered in the tag 202 is read by a reader 203 and is forwarded to the computer 150. In order to easily read the tag 202 when the retainer 200 is attached to the attachment section 201, the reader 203 may be set in the attachment section 201. For example, the tag 202 is a bar code, and the reader 203 is a bar code reader.

(Driver 130)

The driver 130 changes the relative position between the subject 100 and the receiver 120. In this embodiment, the driver 130 is a device that moves the supporter 122 in the XY direction and is an electric XY stage equipped with a stepping motor. The driver 130 includes a motor, such as a stepping motor, for generating a driving force, a driving mechanism that transmits the driving force, and a position sensor that detects positional information of the receiver 120. The driving mechanism used may be, for example, a lead screw mechanism, a link mechanism, a gear mechanism, or a hydraulic mechanism. The position sensor used may be, for example, a potentiometer that uses an encoder or a variable resistor.

The driver 130 is not limited to the type that changes the relative position between the subject 100 and the receiver 120 in the XY (two-dimensional) direction, and may change the relative position one-dimensionally or three-dimensionally. With regard to the movement path, scanning may be performed planarly in a spiral pattern or in a line-and-space fashion, or the movement path may be inclined to three-dimensionally conform to the surface of the body. The probe 180 may be moved so as to maintain a fixed distance from the surface of the subject 100. In this case, the driver 130 may measure the amount of movement of the probe by, for example, monitoring the rotation speed of the motor.

The driver 130 may fix the receiver 120 and move the subject 100 so long as the relative position between the subject 100 and the receiver 120 can be changed. If the subject 100 is to be moved, a configuration that moves the subject 100 by moving the retainer that retains the subject 100 is conceivable. Alternatively, the subject 100 and the receiver 120 may both be moved.

The driver 130 may move the relative position continuously or may move the relative position in a step- and-repeat fashion. The driver 130 may be an electric stage that moves the relative position along a programmed path, or may be a manual stage. Specifically, the modality device according to this embodiment may be not equipped with the driver 130 and may be of a handheld type in which the user holds and operates the probe 180.

In this embodiment, the driver 130 performs scanning by simultaneously driving the light radiator 110 and the receiver 120. Alternatively, the driver 130 may drive the light radiator 110 alone or may drive the receiver 120 alone.

(Signal Collector 140)

The signal collector 140 includes an amplifier that amplifies electric signals, which are analog signals, output from the transducers 121, and also includes an A/D converter that converts the analog signals output from the amplifier into digital signals. The signal collector 140 may be constituted by, for example, an FPGA (field programmable gate array) chip. The digital signals output from the signal collector 140 are stored in a storage unit 152 within the computer 150. The signal collector 140 is also called a data acquisition system (DAS). The concept of an electric signal in this description is that the electric signal includes both an analog signal and a digital signal. Alternatively, the signal collector 140 may be connected to a photo sensor attached to the light emitter of the light radiator 110 and may start a process in synchronization with an output of light from the light radiator 110. As another alternative, the signal collector 140 may start the process in synchronization with a command given by using, for example, a freeze button.

(Computer 150)

The computer 150 as an information processing apparatus includes an arithmetic unit 151, the storage unit 152, and a controller 153. The functions of the respective components will be described in the description of the processing flow.

Units having the role of an arithmetic function and serving as the arithmetic unit 151 may be constituted by a processor, such as a CPU or a GPU (graphics processing unit), and an arithmetic circuit, such as an FPGA (field programmable gate array) chip. These units may be constituted by a single processor and arithmetic circuit or by a plurality of processors and arithmetic circuits. The arithmetic unit 151 may receive various types of parameters, such as the sound velocity of the subject and the configuration of the retainer, from the input unit 170 and may process a reception signal.

The storage unit 152 may be constituted by a non-transitory storage medium, such as a ROM (read-only memory), a magnetic disk, or a flash memory. Moreover, the storage unit 152 may be a volatile medium, such as a RAM (random access memory). A storage medium that stores a program is a non-transitory storage medium. The storage unit 152 may be constituted by a single storage medium or by a plurality of storage media.

The storage unit 152 can store image data indicating a photoacoustic image generated by the arithmetic unit 151 in accordance with a method to be described below.

The controller 153 is constituted by an arithmetic element, such as a CPU. The controller 153 controls the operation of each component of the modality device. The controller 153 may receive command signals given by various types of operations, such as a command for starting a measuring process, from the input unit 170 so as to control the components of the modality device. Furthermore, the controller 153 reads a program code stored in the storage unit 152 and controls the operation of each component of the modality device.

The computer 150 may be a dedicatedly-designed workstation. The components of the computer 150 may be constituted by different hardware units. Moreover, at least one of the components of the computer 150 may be constituted by a single hardware unit.

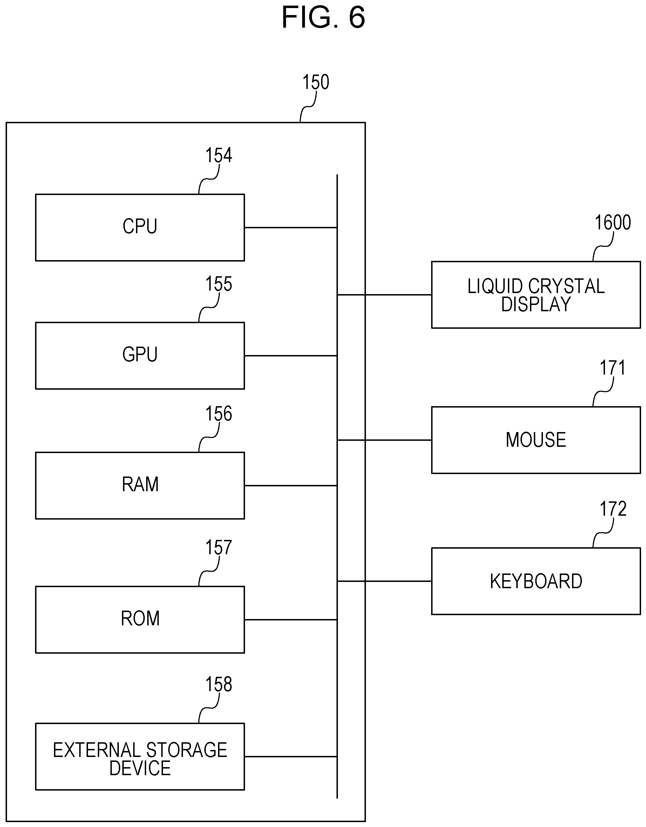

Fig. 6 illustrates a specific configuration example of the computer 150 according to this embodiment. The computer 150 according to this embodiment is constituted by a CPU 154, a GPU 155, a RAM 156, a ROM 157, and an external storage device 158. The computer 150 is connected to a liquid crystal display 161 as the display unit 160 and to a mouse 171 and keyboard 172 as the input unit 170.

The computer 150 and the plurality of transducers 121 may be provided by being accommodated within a common housing. Alternatively, some signal processing may be performed in the computer accommodated in the housing, while the remaining signal processing may be performed in a computer provided outside the housing. In this case, the computers provided inside and outside the housing may collectively be referred to as a computer according to this embodiment. In other words, the hardware constituting the computer does not have to be accommodated within a single housing.

(Display Unit 160)

The display unit 160 is a display, such as a liquid crystal display, an organic EL (electroluminescence) FED, an eyeglasses-type display, or a head mount display, and displays an image based on volume data obtained by the computer 150 or a numerical value of a specific position. The display unit 160 may also display a GUI used for displaying an image based on volume data or for operating a device. When subject information is to be displayed, the subject information may be displayed after image processing (such as an adjustment of a brightness value) is performed in the display unit 160 or the computer 150. The display unit 160 may be provided separately from the modality device. The computer 150 can transmit photoacoustic image data to the display unit 160 in a wired or wireless manner.

(Input Unit 170)

The input unit 170 used may be a control console operable by a user and constituted by, for example, a mouse and a keyboard. Alternatively, the display unit 160 may be constituted by a touchscreen, such that the display unit 160 may be used as the input unit 170.

The input unit 170 may be configured to receive information about the position and the depth to be observed. The input method may involve inputting a numerical value or inputting information by operating a slider bar. Furthermore, the image displayed on the display unit 160 may be updated in accordance with the input information. Accordingly, a user can set an appropriate parameter while checking an image generated in accordance with the parameter set by the user.

The components of the modality device may be constituted by separate devices, or may be constituted by a single integrated device. Furthermore, at least one of the components of the modality device may be constituted by a single integrated device.

Furthermore, information to be exchanged between the components of the modality device may be exchanged in a wired or wireless manner.

(Subject 100)

The subject 100 does not constitute the modality device, but will be described below. The modality device according to this embodiment can be used for the purpose of diagnosing or monitoring a chemical treatment of, for example, a malignant tumor or a blood vessel disease in a human or an animal. Therefore, it is assumed that the subject 100 is a target diagnostic site, such as a breast, an organ, a vascular network, a head region, a neck region, an abdominal region, and extremities including fingers and toes of a biological organism, specifically, a human or an animal. For example, if a human body is to be photographed by using a photoacoustic device, the light absorber may be, for example, oxyhemoglobin or deoxyhemoglobin, a blood vessel containing a large amount thereof, or a new blood vessel formed near a tumor. Alternatively, the light absorber may be, for example, a plaque on a carotid wall. Furthermore, the light absorber may be a pigment, such as methylene blue (MB) or indocyanine green (ICG), fine gold particles, or an externally introduced material obtained by collecting or chemically modifying the pigment and the fine gold particles. If a human body is to be imaged using MRA, CTA, or contrast-enhanced ultrasonic echo, a contrast agent, as typified by a gadolinium contrast agent, an iodinated contrast agent, and a perflubutane microbubble contrast agent, may be used.

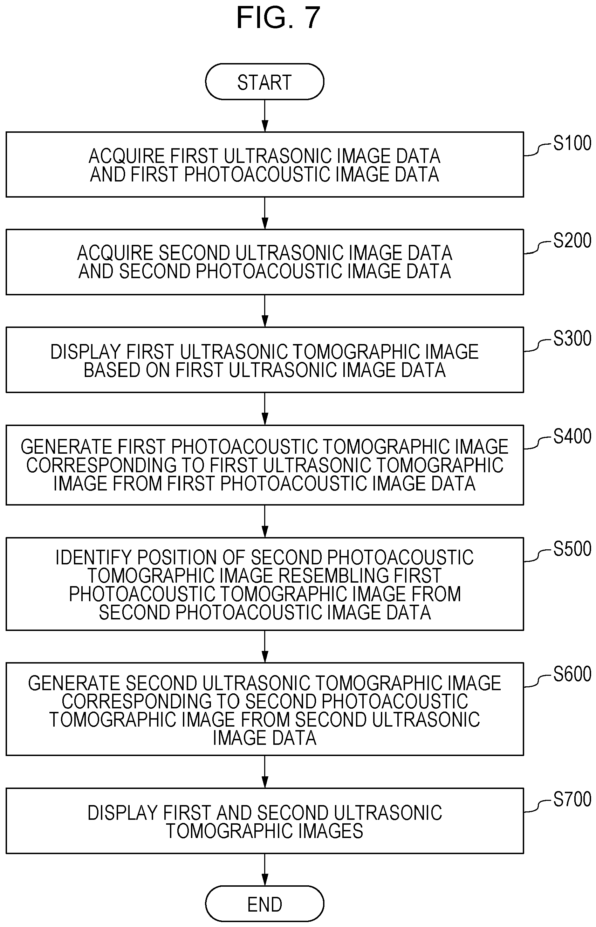

Next, an image display method including information processing according to this embodiment will be described with reference to Fig. 7. The steps are executed by the computer 150 controlling the operation of each component of the modality device.

(S100: Step for Acquiring First Ultrasonic Image Data and First Photoacoustic Image Data)

The modality device according to this embodiment photographs the subject 100 before a treatment so as to generate ultrasonic image data 11 and photoacoustic image data 12, and stores the image data in the storage unit 152. Because the ultrasonic image data 11 and the photoacoustic image data 12 are generated at the same time before a treatment in this embodiment, the coordinate systems of the two pieces of image data correspond with each other. Specifically, each piece of image data corresponds with the positional relationship between the device coordinate system of the modality device and the subject 100. In this embodiment, ultrasonic image data obtained before a treatment is referred to as first ultrasonic image data (first volume data). Furthermore, photoacoustic image data obtained before a treatment is referred to as first photoacoustic image data (second volume data). A method of how the modality device according to this embodiment generates image data will be described below.

First, the user uses the input unit 170 to designate control parameters, such as the irradiation conditions (e.g., cyclic frequency and wavelength) of the light radiator 110 necessary for acquiring subject information and the position of the probe 180. The computer 150 sets the control parameters set based on the command of the user. Alternatively, the computer 150 may set control parameters set in advance.

Based on the set control parameters, the controller 153 causes the driver 130 to move the probe 180 to the designated position. If imaging at a plurality of positions is designated, the driver 130 first moves the probe 180 to a first designated position. The driver 130 may move the probe 180 to a preliminarily-programmed position when a measurement start command is given. In a case of a handheld type, the user may hold the probe 180 and move the probe 180 to a desired position.

The light radiator 110 radiates light onto the subject 100 based on the control parameters designated by the user. Light generated from the light source 111 is radiated onto the subject 100 as pulsed light via the optical system 112. Then, the pulsed light is absorbed within the subject 100, and a photoacoustic wave occurs due to a photoacoustic effect. In addition to transmitting the pulsed light, the light radiator 110 transmits a synchronization signal to the signal collector 140.

When the signal collector 140 receives the synchronization signal transmitted from the light radiator 110, the signal collector 140 starts to perform signal collecting operation. Specifically, the signal collector 140 performs amplification and A/D conversion on an analog electric signal output from the receiver 120 and derived from a photoacoustic wave, so as to generate an amplified digital electric signal, and outputs the signal to the computer 150. The computer 150 stores the signal transmitted from the signal collector 140 into the storage unit 152. The computer 150 can acquire photoacoustic signal data in this manner.

While the modality device generates the digital signal derived from the photoacoustic wave, the receiver 120 exchanges an ultrasonic wave with the subject 100 so as to output an analog electric signal derived from an ultrasonic echo. In addition to transmitting the ultrasonic wave, the receiver 120 transmits a synchronization signal to the signal collector 140.

When the signal collector 140 receives the synchronization signal transmitted from the receiver 120, the signal collector 140 starts to perform signal collecting operation. Specifically, the signal collector 140 performs amplification and A/D conversion on the analog electric signal output from the receiver 120 and derived from the ultrasonic echo, so as to generate an amplified digital electric signal, and outputs the signal to the computer 150. The computer 150 stores the signal transmitted from the signal collector 140 into the storage unit 152. The computer 150 can acquire ultrasonic echo signal data in this manner. The exchanging of an ultrasonic wave is preferably performed at a timing at which the reception of a photoacoustic wave is not interfered. Therefore, the exchanging of an ultrasonic wave may be performed between the irradiation of pulsed light for exciting a photoacoustic wave and the next light irradiation.

If imaging at a plurality of scan positions is set, the aforementioned irradiation of pulsed light at each of the scan positions, the aforementioned exchanging of an ultrasonic wave, and the aforementioned generation of a digital signal derived from an acoustic wave are repeated.

The arithmetic unit 151 in the computer 150 generates first photoacoustic image data as volume data based on the photoacoustic signal data stored in the storage unit 152, and stores the first photoacoustic image data in the storage unit 152. Moreover, the arithmetic unit 151 generates first ultrasonic image data as volume data based on ultrasonic signal data stored in the storage unit, and stores the first ultrasonic image data in the storage unit 152.

A reconfiguration algorithm used for converting signal data into volume data as a three-dimensional spatial distribution may be any method, such as a time-domain-based back-projection method, a Fourier-domain-based back-projection method, or a model-based method (repetitive calculation method). Examples of the time-domain-based back-projection method include the universal back-projection (UBP) method, the filtered back-projection (FBP) method, and the delay-and-sum method. For example, the arithmetic unit 151 may use the UBP method expressed by expression (1) as a reconfiguration method for acquiring, as photoacoustic image data, a three-dimensional spatial distribution of a generated sound pressure (initial sound pressure) of an acoustic wave.

$$p_0(\mathbf{r}_0) = \frac{\sum_{i}^{N} b\left(\mathbf{r}_i, t = \frac{|\mathbf{r}_i - \mathbf{r}_0|}{c}\right) \cdot \Delta\Omega_i}{\sum_{i}^{N} \Delta\Omega_i}$$

$$b(\mathbf{r}, t) = 2p(\mathbf{r}, t) - 2t\frac{\partial p(\mathbf{r}, t)}{\partial t} \qquad (1)$$

In this case, $r_0$ denotes a position vector indicating a position where reconfiguration is to be performed (also called a reconfiguration position or a position of interest), $p_0(r_0, t)$ denotes an initial sound pressure at the reconfiguration position, and c denotes a sound velocity on a propagation path. Furthermore, $\Delta\Omega_i$ denotes a solid angle from the reconfiguration position to an i-th transducer 121, and N denotes the number of transducers 121 to be used for the reconfiguration. Expression (1) indicates that phasing addition (back-projection) is performed by performing, for example, differentiation on a reception signal $p(r_i, t)$ and multiplying the signal by the weight of the solid angle. In expression (1), t denotes a time (propagation time) in which a photoacoustic wave propagates along an acoustic ray connecting the position of interest and each transducer 121. In the calculation of $b(r_i, t)$, another arithmetic process may be performed. Examples include frequency filtering (e.g., low pass, high pass, band pass), deconvolution, envelope detection, and wavelet filtering.

The arithmetic unit 151 may calculate a light fluence distribution within the subject 100 when light is radiated onto the subject 100, and may divide an initial sound pressure distribution by the light fluence distribution so as to acquire absorption-coefficient-distribution information. In this case, the absorption-coefficient-distribution information may be acquired as photoacoustic image data. The computer 150 can calculate a light-fluence spatial distribution within the subject 100 in accordance with a method of numerically solving a transport equation or a diffusion equation expressing the behavior of light energy in a medium that absorbs and scatters light. The numerically solving method used may be, for example, the finite element method, the finite difference method, or the Monte Carlo method. For example, the computer 150 may solve a light diffusion equation indicated in expression (2) so as to calculate a light-fluence spatial distribution within the subject 100.

$$\frac{1}{c}\frac{\partial}{\partial t}\Phi(r, t) = -\mu_a\Phi(r, t) + \nabla \cdot (D\nabla\Phi(r, t)) + S(r, t)$$

... expression (2)

In this case, D denotes a diffusion coefficient, $\mu_a$ denotes an absorption coefficient, S denotes an input intensity of radiated light, $\phi$ denotes an incoming light fluence, r denotes a position, and t denotes time.

Furthermore, step S300 and step S400 may be performed by using light beams of a plurality of wavelengths, and the arithmetic unit 151 may acquire absorption-coefficient-distribution information corresponding to each of the light beams of the plurality of wavelengths. Then, based on the absorption-coefficient-distribution information corresponding to each of the light beams of the plurality of wavelengths, the arithmetic unit 151 may acquire, as photoacoustic image data, spatial distribution information serving as spectral information and indicating the concentration of the material constituting the subject 100. Specifically, the arithmetic unit 151 may acquire spectral information by using signal data corresponding to the light beams of the plurality of wavelengths.

This embodiment relates to an example where the modality device generates image data so that the computer 150 acquires the image data. Alternatively, the computer 150 may read image data stored in the storage unit so as to acquire the image data. For example, the computer 150 as volume-data acquiring means may acquire volume data generated in advance in the modality device by reading the volume data from a storage unit, such as a PACS (picture archiving and communication system). Accordingly, the information processing method according to the present invention can also be applied to preliminarily-generated volume data.

(S200: Step for Acquiring Second Ultrasonic Image Data and Second Photoacoustic Image Data)

The modality device according to this embodiment photographs the subject 100 after the treatment so as to generate ultrasonic image data 21 and photoacoustic image data 22, and stores the image data in the storage unit 152. Because the ultrasonic image data 21 and the photoacoustic image data 22 are generated at the same time after the treatment in this embodiment, the coordinate systems of the two pieces of image data correspond with each other. Specifically, each piece of image data corresponds with the positional relationship between the device coordinate system of the modality device and the subject 100. In this embodiment, the ultrasonic image data 21 obtained after the treatment is referred to as second ultrasonic image data (third volume data). Furthermore, the photoacoustic image data obtained after the treatment is referred to as second photoacoustic image data (fourth volume data). The method for generating the image data is the same as the method described in step S100.

(S300: Step for Displaying First Ultrasonic Tomographic Image Based on First Ultrasonic Image Data)

The computer 150 as tomographic-image acquiring means generates a tomographic image 11a based on the ultrasonic image data 11, and stores the tomographic image 11a in the storage unit 152. Then, the computer 150 as display control means causes the display unit 160 to display the tomographic image 11a. In this embodiment, the tomographic image 11a generated based on the pre-treatment ultrasonic image data 11 is referred to as a first ultrasonic tomographic image (or a first tomographic image).

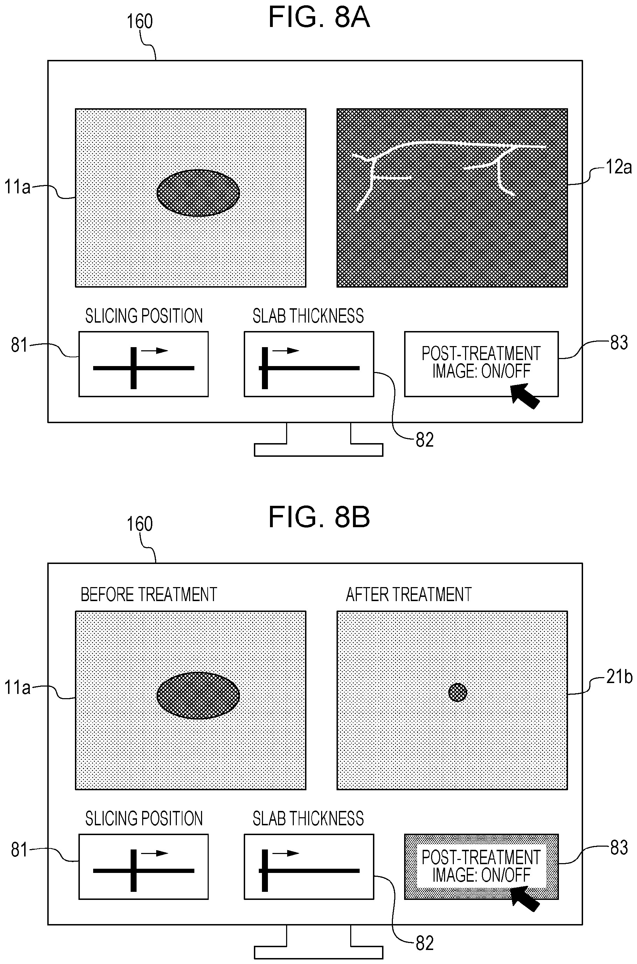

Fig. 8 illustrates a GUI displayed on the display unit 160 according to this embodiment. The display unit 160 displays, as a GUI, a slider 81 used for adjusting a slicing position based on a command of the user, a slider 82 used for adjusting a slab thickness based on a command of the user, and an item 83 used for switching between display on and off modes of a post-treatment tomographic image. Fig. 8A illustrates a display screen of a display mode when the post-treatment tomographic image is not displayed. Fig. 8B illustrates a display screen of a display mode when the post-treatment tomographic image is displayed.

The display unit 160 in Fig. 8A displays the tomographic image 11a generated in accordance with the slicing position and the slab thickness set based on a command of the user. The tomographic image 11a is a tomographic image of the pre-treatment ultrasonic image data 11 shown in Fig. 2. In this case, the user can search through the ultrasonic image data 11 for a tomographic image depicting a tumor while operating the sliders 81 and 82.

The tomographic image in this embodiment may be a group of voxels located in a specific plane or may be an image obtained by rendering a voxel group in a certain region. The rendering method used may be any known method, such as the maximum intensity projection (MIP) method, the minimum intensity projection (MinIP) method, the ray sum method, the average-value projection method, the median-value projection method, the volume rendering method, and the surface rendering method. In the example shown in Fig. 8, the tomographic image is an image obtained by rendering a voxel group corresponding to a slab thickness set using the slider 82, with reference to a slicing position set using the slider 81.

(S400: Step for Generating First Photoacoustic Tomographic Image Corresponding to First Ultrasonic Tomographic Image from First Photoacoustic Image Data)

The computer 150 generates a tomographic image 12a corresponding to the tomographic image 11a from the photoacoustic image data 12 and stores the tomographic image 12a. In this embodiment, the tomographic image 12a of the pre-treatment photoacoustic image data 12 corresponding to the tomographic image 11a is referred as a first photoacoustic tomographic image (second tomographic image).

In Fig. 8A, the tomographic image 11a of the ultrasonic image data 11 and the tomographic image 12a of the photoacoustic image data 12 are displayed in a side-by-side fashion. The display cross sections of the tomographic image 11a and the tomographic image 12a are set in correspondence with each other, such that images of the same position are depicted. However, due to being different image types, the tomographic image 11a mainly depicts a tumor, whereas the tomographic image 12a mainly depicts a blood vessel.

Although this embodiment relates to an example where the computer 150 displays the tomographic image 11a and the tomographic image 12a in a side-by-side fashion, the images may be displayed in a superposed fashion or in a switching fashion. Furthermore, if the doctor desires to observe a tumor image alone, the computer 150 may cause the display unit 160 to display the tomographic image 11a alone and to cause the display unit 160 not to display the tomographic image 12a.

Furthermore, although this embodiment relates to an example where the tomographic image 11a and the tomographic image 12a are generated from volume data, the computer 150 may acquire the tomographic image 11a and the tomographic image 12a without the intervention of the volume data. For example, the computer 150 may generate the tomographic image 11a and the tomographic image 12a from signal data, or may acquire preliminarily-generated tomographic image 11a and tomographic image 12a by reading the images from the storage unit 152.

(S500: Step for Identifying Position of Second Photoacoustic Tomographic Image Resembling First Photoacoustic Tomographic Image from Second Photoacoustic Image Data)

The computer 150 as positional-information acquiring means identifies the tomographic image 22b resembling the tomographic image 12a from the post-treatment photoacoustic image data 22 and acquires information indicating the position of the tomographic image. In this embodiment, the tomographic image 22b resembling the tomographic image 12a is referred to as a second photoacoustic tomographic image (third tomographic image).

For example, the computer 150 may calculate similarities between the tomographic image 12a and images of a plurality of cross sections of the photoacoustic image data 22, and may acquire information indicating the position of a cross section with a high similarity. The computer 150 may set a tomographic image of the photoacoustic image data 22 when the similarity is higher than a predetermined threshold value as a tomographic image resembling the tomographic image 12a. Moreover, the computer 150 may calculate similarities between the tomographic image 12a and a plurality of tomographic images of the photoacoustic image data 22, and may set a tomographic image with the highest similarity as a tomographic image resembling the tomographic image 12a.

For example, a function used for calculating a similarity may be any similarity scale, such as SSD (sum of squared difference), SAD (sum of absolute difference), mutual information, and cross correlation. Furthermore, for example, a similarity function may be acquired by extracting a characteristic form from the photoacoustic image data and measuring the degree of coincidence with these positions. The characteristics to be extracted may be anatomical characteristics of, for example, a blood vessel or characteristics extracted in accordance with a known technique commonly used in the image processing field, such as edge detection or corner detection. Moreover, the characteristics to be extracted may be higher-order local image characteristics, such as SIFT characteristics and SURF characteristics, commonly used in the technical field of, for example, computer visions. According to these methods, it is possible to acquire a similarity function that is more robust against a difference in the brightness distribution between pieces of image data and against inclusion of noise.

Furthermore, by causing the display unit 160 to display the tomographic image 12a and to display the tomographic image 22a of the photoacoustic image data 22 next thereto and allowing the user to change the slicing position of the photoacoustic image data 22 by using the slider 81, the tomographic image 22b as an image resembling the tomographic image 12a may be searched for and be set. Specifically, the computer 150 may acquire information indicating the position of the tomographic image 22b based on a command of the user.

With regard to an image of each cross section of the photoacoustic image data 22 to be compared with the tomographic image 12a, the image may be obtained by rendering a voxel group of a certain region. In that case, the image of each cross section of the photoacoustic image data 22 may be an image obtained by rendering a volume that is about the same as the volume when the tomographic image 12a is generated.

Moreover, the computer 150 may generate base images ($\phi$) describing the characteristics of a blood vessel image from the photoacoustic image data 12 and the photoacoustic image data 22 and compare the base images, so as to identify a cross section that resembles the tomographic image 12a from the photoacoustic image data 22. Specifically, the computer 150 may generate the tomographic image 12a as a base image describing the characteristics of a blood vessel image from the photoacoustic image data 12. Then, the computer 150 may generate a base image describing the characteristics of a blood vessel image from the photoacoustic image data 22 and compare the generated base image with the tomographic image 12a as a base image, so as to identify a cross section, resembling the tomographic image 12a, where the base image in the photoacoustic image data 22 is located. Alternatively, the computer 150 may generate a base image describing the characteristics of a blood vessel image from the tomographic image 12a and compare the generated base image with the photoacoustic image data 22 as a base image. Examples of the base-image generating method include a principal component analysis, an independent principal component analysis, nonnegative matrix factorization, and sparse signal decomposition. If a base image describing the characteristics of an image can be obtained by any of these methods, a blood vessel image $P_{0,vessel}$ obtained from photoacoustic image data can be approximately expressed with expression (3) by using the base image.

$$p_{0,vessel} = a_0\phi_0 + a_1\phi_1 + a_2\phi_2 + \ldots + a_n\phi_n = \sum_{i=0}^{n} a_i\phi_i$$

$$\cdots (3)$$

In this case, ai (i = 0, ..., n) denotes a coefficient at each base. Assuming that a base matrix obtained by arranging the obtained bases is defined as $\Phi = (\phi_0, ..., \phi_n)$ and a coefficient vector obtained by arranging the obtained coefficients is defined as a = (a0, a1, ..., an), expression (3) can be expressed as $P_{0,vessel} = \Phi_a$ by using a matrix.

(S600: Step for Generating Second Ultrasonic Tomographic Image Corresponding to Second Photoacoustic Tomographic Image from Second Ultrasonic Image Data)

The computer 150 generates the tomographic image 21b corresponding to the tomographic image 22b from the ultrasonic image data 21 based on the information indicating the position of the tomographic image 22b, and stores the tomographic image 21b. In this case, the computer 150 may acquire and store information indicating the position of the tomographic image 21b. In this embodiment, the tomographic image 21b of the post-treatment ultrasonic image data 21 corresponding to the tomographic image 22b is referred to as a second ultrasonic tomographic image (fourth tomographic image). Accordingly, the post-treatment tomographic image 21b depicting the same lesion as the pre-treatment tomographic image 11a can be generated.

(S700: Step for Displaying First and Second Ultrasonic Tomographic Images)

As shown in Fig. 8B, the computer 150 causes the display unit 160 to display an image including the tomographic image 11a and the tomographic image 21b. Because the tomographic image 11a and the tomographic image 21b depict the same lesion, the displaying of the image including both of these images in this manner allows the doctor to temporally observe the lesion.

In this embodiment, the user operates the item 83 by using the input unit 170 in the state shown in Fig. 8A so as to switch the state to the state in Fig. 8B. Specifically, the computer 150 can switch the display mode between display on and off modes of the post-treatment tomographic image 21b based on a command of the user. Moreover, the computer 150 may switch the display mode between display on and off modes of the pre-treatment tomographic image 11a based on a command of the user when the post-treatment tomographic image 21b is displayed. Furthermore, the computer 150 may cause the display unit 160 to display the post-treatment tomographic image 21b and the post-treatment tomographic image 22b using at least one of display methods including a side-by-side display method, a superposed display method, and a switching display method.

Although this step relates to an example where the tomographic image 22b is displayed, the computer 150 may acquire the information indicating the position of the tomographic image 22b and store the information in the storage unit 152. In other words, in this embodiment, the tomographic image 22b does not have to be displayed. Furthermore, since the position of the tomographic image 22b can be identified from the position of a tomographic image 12b, the computer 150 may acquire information indicating the position of the tomographic image 12b and store the information in the storage unit 152, and does not have to acquire the information indicating the position of the tomographic image 22b.

Although the above description relates to an example where a cross section including a resembling tomographic image is searched for by comparing two-dimensional tomographic images, a resembling three-dimensional region may be searched for by comparing three-dimensional images generated from photoacoustic image data. For example, the computer 150 may generate pre-treatment and post-treatment three-dimensional blood vessel images as base images describing the characteristics of a blood vessel image from the pre-treatment photoacoustic image data 12 and the post-treatment photoacoustic image data 22, respectively. Then, the computer 150 may perform a deformation process for expanding, rotating, and shifting at least one of the three-dimensional blood vessel images so as to positionally align the pre-treatment and post-treatment three-dimensional blood vessel images with each other, and may acquire coordinate deformation parameters thereof. For example, the computer 150 can acquire, as a coordinate deformation parameter, information about a deformation field constituted by a displacement vector extending from each voxel position of one of the three-dimensional images toward a corresponding voxel position of the other three-dimensional image. Moreover, the computer 150 may use the coordinate deformation parameter to perform a conversion (i.e., a deformation process) on the coordinate system of at least one of the pre-treatment ultrasonic image data 21 and the post-treatment ultrasonic image data 22. By performing this process, the coordinate systems that depict a lesion can be made substantially the same in the ultrasonic image data 11 and the ultrasonic image data 21. The computer 150 may generate pre-treatment and post-treatment tomographic images corresponding to a cross section at the same coordinates respectively from the ultrasonic image data 11 and the ultrasonic image data 21 having undergone the deformation process, and may display the tomographic images. Specifically, the tomographic image 11a depicting a tumor in the ultrasonic image data 11 and a tomographic image located at the same coordinates as the tomographic image 11a and obtained from volume data obtained by performing the deformation process on the ultrasonic image data 21 can depict a tumor or a lesion. Although the above description relates to an example where three-dimensional blood vessel images are generated and compared, the three-dimensional images to be compared do not have to be blood vessel images.

Although this embodiment relates to an example where an image with which a lesion can be identified from ultrasonic image data is generated by referring to images resembling each other between pieces of photoacoustic image data, the embodiment can also be applied to volume data obtained in another modality device, as mentioned above.

(Other Embodiments)

The present invention is also realized by executing the following process. Specifically, the process involves supplying software (program) for realizing the functions of the above-described embodiment to a system or an apparatus via a network or any of various types of storage media, and causing a computer (such as a CPU or an MPU) in the system or the apparatus to read and execute the program.

The present invention is not limited to the above-described embodiments, and various modifications and alterations are possible so long as they do not depart from the spirit and scope of the invention. Accordingly, the following claims are attached for publicizing the scope of the invention.

This application claims the benefit of Japanese Patent Application No. 2017-118013 filed June 15, 2017, which is hereby incorporated by reference herein in its entirety.

**Claims**

1. An information processing apparatus comprising:

    volume-data acquiring means for acquiring first volume data and second volume data whose coordinate systems correspond with each other and that are of different image types, acquiring third volume data of an image type identical to that of the first volume data and whose coordinate system is different from that of the first volume data, and acquiring fourth volume data of an image type identical to that of the second volume data and whose coordinate system corresponds with that of the third volume data;
    tomographic-image generating means for generating a first tomographic image corresponding to a cross section set based on a command of a user from the first volume data, and generating a second tomographic image corresponding to the first tomographic image from the second volume data;
    positional-information acquiring means for identifying a third tomographic image resembling the second tomographic image from the fourth volume data, and acquiring information indicating a position of the third tomographic image; and
    display control means,
    wherein the tomographic-image generating means generates a fourth tomographic image corresponding to the third tomographic image from the third volume data based on the information indicating the position of the third tomographic image, and
    wherein the display control means causes display means to display an image including the fourth tomographic image.

2. The information processing apparatus according to Claim 1,
   wherein each of the first volume data and the third volume data is volume data containing an image of a tumor or an inflammatory site.

3. The information processing apparatus according to Claim 1,
   wherein each of the second volume data and the fourth volume data is volume data containing an image of at least one of a blood vessel, a lymph vessel, and a skin.

4. The information processing apparatus according to Claim 1,
wherein each of the first volume data and the third volume data is ultrasonic image data, MRI image data, or CT image data.

5. The information processing apparatus according to Claim 1,
wherein each of the second volume data and the fourth volume data is photoacoustic image data, MRA image data, CTA image data, or contrast-enhanced ultrasonic image data.

6. The information processing apparatus according to Claim 1,
wherein the display control means causes the display means to display an image including the first tomographic image and the fourth tomographic image.

7. The information processing apparatus according to any one of Claims 1 to 6,
wherein the positional-information acquiring means calculates a similarity between the second tomographic image and tomographic images of a plurality of cross sections of the fourth volume data, and acquires the information indicating the position of the third tomographic image based on the similarity.

8. The information processing apparatus according to any one of Claims 1 to 7,
wherein each of the second volume data and the fourth volume data is volume data containing a blood vessel image,
wherein the tomographic-image generating means generates the second tomographic image as a base image describing characteristics of the blood vessel image, and
wherein the positional-information acquiring means identifies the third tomographic image resembling the second tomographic image by comparing the second tomographic image as the base image describing the characteristics of the blood vessel image with a base image generated from the fourth volume data and describing the characteristics of the blood vessel image, and acquires the information indicating the position of the third tomographic image.

9. An information processing apparatus comprising:

image-data acquiring means for acquiring first ultrasonic image data, acquiring first photoacoustic image data whose coordinate system corresponds with that of the first ultrasonic image data, acquiring second ultrasonic image data whose coordinate system is different from that of the first ultrasonic image data, and acquiring second photoacoustic image data whose coordinate system corresponds with that of the second ultrasonic image data;
tomographic-image generating means for generating a first ultrasonic tomographic image corresponding to a cross section set based on a command of a user from the first ultrasonic image data, and generating a first photoacoustic tomographic image corresponding to the first ultrasonic tomographic image from the first photoacoustic image data;
positional-information acquiring means for identifying a second photoacoustic tomographic image resembling the first photoacoustic tomographic image from the second photoacoustic image data, and acquiring information indicating a position of the second photoacoustic tomographic image; and
display control means,
wherein the tomographic-image generating means generates a second ultrasonic tomographic image corresponding to the second photoacoustic tomographic image from the second ultrasonic image data based on the information indicating the position of the second photoacoustic tomographic image, and
wherein the display control means causes display means to display an image including the second ultrasonic tomographic image.

10. The information processing apparatus according to Claim 9,
wherein the display control means causes the display means to display an image including the first ultrasonic tomographic image and the second ultrasonic tomographic image.

11. The information processing apparatus according to Claim 9 or 10,
wherein the positional-information acquiring means calculates a similarity between the first photoacoustic image data and tomographic images of a plurality of cross sections of the first photoacoustic image data, and acquires the information indicating the position of the second photoacoustic tomographic image based on the similarity.

12. The information processing apparatus according to any one of Claims 9 to 11,
wherein the tomographic-image generating means generates the first photoacoustic tomographic image as a base image describing characteristics of a blood vessel image, and
wherein the positional-information acquiring means identifies the second photoacoustic tomographic image resembling the first photoacoustic tomographic image by comparing the first photoacoustic tomographic image as the base image describing the characteristics of the blood vessel image with a base image generated from the second photoacoustic image data and describing the characteristics of the blood vessel image, and acquires the information indicating the position of the second photoacoustic tomographic image.

13. An information processing method comprising:

acquiring first volume data and second volume data whose coordinate systems correspond with each other and that are of different image types;
acquiring third volume data of an image type identical to that of the first volume data and whose coordinate system is different from that of the first volume data;
acquiring fourth volume data of an image type identical to that of the second volume data and whose coordinate system corresponds with that of the third volume data;
generating a first tomographic image corresponding to a cross section set based on a command of a user from the first volume data;
generating a second tomographic image corresponding to the first tomographic image from the second volume data;
identifying a third tomographic image resembling the second tomographic image from the fourth volume data, and acquiring information indicating a position of the third tomographic image;
generating a fourth tomographic image corresponding to the third tomographic image from the third volume data based on the information indicating the position of the third tomographic image, and
causing display means to display an image including the fourth tomographic image.

14. An information processing method comprising:

acquiring first ultrasonic image data;
acquiring first photoacoustic image data whose coordinate system corresponds with that of the first ultrasonic image data;
acquiring second ultrasonic image data whose coordinate system is different from that of the first ultrasonic image data;
acquiring second photoacoustic image data whose coordinate system corresponds with that of the second ultrasonic image data;
generating a first ultrasonic tomographic image corresponding to a cross section set based on a command of a user from the first ultrasonic image data;
generating a first photoacoustic tomographic image corresponding to the first ultrasonic tomographic image from the first photoacoustic image data;
identifying a second photoacoustic tomographic image resembling the first photoacoustic tomographic image from the second photoacoustic image data, and acquiring information indicating a position of the second photoacoustic tomographic image;
generating a second ultrasonic tomographic image corresponding to the second photoacoustic tomographic image from the second ultrasonic image data based on the information indicating the position of the second photoacoustic tomographic image; and
causing display means to display an image including the second ultrasonic tomographic image.

15. A program for causing a computer to execute the information processing method according to Claim 13 or 14.

# FIG. 1A

# FIG. 1B

FIG. 2

# FIG. 3

# FIG. 4

## FIG. 5A

## FIG. 5B

# FIG. 6

# FIG. 7

START

ACQUIRE FIRST ULTRASONIC IMAGE DATA AND FIRST PHOTOACOUSTIC IMAGE DATA — S100

ACQUIRE SECOND ULTRASONIC IMAGE DATA AND SECOND PHOTOACOUSTIC IMAGE DATA — S200

DISPLAY FIRST ULTRASONIC TOMOGRAPHIC IMAGE BASED ON FIRST ULTRASONIC IMAGE DATA — S300

GENERATE FIRST PHOTOACOUSTIC TOMOGRAPHIC IMAGE CORRESPONDING TO FIRST ULTRASONIC TOMOGRAPHIC IMAGE FROM FIRST PHOTOACOUSTIC IMAGE DATA — S400

IDENTIFY POSITION OF SECOND PHOTOACOUSTIC TOMOGRAPHIC IMAGE RESEMBLING FIRST PHOTOACOUSTIC TOMOGRAPHIC IMAGE FROM SECOND PHOTOACOUSTIC IMAGE DATA — S500

GENERATE SECOND ULTRASONIC TOMOGRAPHIC IMAGE CORRESPONDING TO SECOND PHOTOACOUSTIC TOMOGRAPHIC IMAGE FROM SECOND ULTRASONIC IMAGE DATA — S600

DISPLAY FIRST AND SECOND ULTRASONIC TOMOGRAPHIC IMAGES — S700

END

# FIG. 8A

160

11a

12a

SLICING POSITION

SLAB THICKNESS

81

POST-TREATMENT
IMAGE: ON/OFF

83

82

# FIG. 8B

160

BEFORE TREATMENT

AFTER TREATMENT

11a

21b

SLICING POSITION

SLAB THICKNESS

81

POST-TREATMENT
IMAGE: ON/OFF

83

82

## INTERNATIONAL SEARCH REPORT

| International application No. |
| --- |
| PCT/JP2018/021670 |

**A. CLASSIFICATION OF SUBJECT MATTER**
Int.Cl. A61B8/14(2006.01)i, A61B5/055(2006.01)i, A61B6/03(2006.01)i,
A61B8/13(2006.01)i

According to International Patent Classification (IPC) or to both national classification and IPC

**B. FIELDS SEARCHED**

Minimum documentation searched (classification system followed by classification symbols)
Int.Cl. A61B8/14, A61B5/055, A61B6/03, A61B8/13

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2010-286472 A (TOSHIBA CORP.) 24 December 2010, entire text, all drawings & US 2010/0289813 A1 & CN 101884545 A | 1–15 |
| A | JP 2009-112468 A (TOSHIBA CORP.) 28 May 2009, entire text, all drawings (Family: none) | 1–15 |
| A | US 2014/0355855 A1 (MIAO, Shun) 04 December 2014, entire text, all drawings (Family: none) | 1–15 |
| A | JP 2015-146989 A (CANON INC.) 20 August 2015, entire text, all drawings & US 2015/0228093 A1 | 1–15 |

☐ Further documents are listed in the continuation of Box C.    ☐ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| 12 July 2018 (12.07.2018) | 24 July 2018 (24.07.2018) |

| Name and mailing address of the ISA/ <br> Japan Patent Office <br> 3-4-3, Kasumigaseki, Chiyoda-ku, <br> Tokyo 100-8915, Japan | Authorized officer <br><br> Telephone No. |
| --- | --- |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7065146 A
- JP 2017118013 A